# EUROPÄISCHE PATENTANMELDUNG

(11) **EP 1 520 536 A1**
(43) Veröffentlichungstag der Anmeldung: **06.04.2005**
(21) Anmeldenummer: 03022056.0
(22) Anmeldetag: 01.10.2003
(51) Int. Cl.: A61B 17/22, A61N 7/00

(54) **Vorrichtung zur Applikation von akustischen Stosswellen**

(71) Anmelder: HMT High Medical Technologies AG, 8574 Lengwil (CH)
(72) Erfinder: Simnacher, Erwin, 78479 Reichenau (DE); van Rijn, Dick, 88145 Hergatz (DE)

(57) **Zusammenfassung**

Vorrichtung, insbesondere Therapievorrichtung zur Applikation von akustischen Stosswellen, die auf ein zu behandelndes Gebiet eines menschlichen oder tierischen Körpers gerichtet sind, bestehend aus einem Gehäuse mit einer Koppelfläche für die Einkopplung der Stosswellen in den zu behandelnden Körper, mit einer Stosswellenerzeugungseinrichtung und einem zur Fokussierung der Stosswellen geeigneten Mittel bei der die Stosswellenerzeugungseinrichtung zusammen mit dem zur Fokussierung der Stosswellen geeigneten Mittel im Gehäuse relativ zur Koppelfläche beweglich ausgebildet ist.

## Beschreibung

Die Erfindung betrifft eine Vorrichtung, insbesondere eine Therapievorrichtung zur Applikation von akustischen Stosswellen gemäss dem Oberbegriff des Anspruchs 1.

Derartige Therapievorrichtungen zur Applikation von akustischen Stosswellen werden beispielsweise für die Lithotripsie oder in der Orthopädie für Humananwendungen eingesetzt. Ebenso kommen die Therapievorrichtungen in der Veterinärmedizin zum Einsatz.

Hierbei wird der zu behandelnde Bereich lokalisiert und eine erforderliche Anzahl akustischer Stosswellen appliziert. Der zu therapierende Bereich kann dabei unterschiedlich weit von der Körperoberfläche des Patienten entfernt liegen. Dieser Abstand hängt einerseits von der Art des zu therapierenden Bereiches (Nierensteine, Weichteilstrukturen), andererseits von der Konstitution des Patienten (adipös/nicht adipös) ab.
Die Eindringtiefe der akustischen Stosswellen wird durch den Ort der Stosswellenerzeugung und der Geometrie der Stosswellenfokussierungseinrichtung definiert.

Zur Positionierung des Stosswellenfokus in die jeweiligen verschieden tief gelegenen Therapiegebiete im Körper des Patienten sind Therapievorrichtungen mit verschieden festen oder variablen Eindringtiefen erforderlich.

Aus DE 195 09 004 C1 ist es bekannt, bei einer Vorrichtung der genannten Art austauschbare Koppelaufsätze wie beispielsweise Ankoppelpolster oder Koppelkissen zur Variierung des Abstandes zwischen der Stosswellenerzeugungseinrichtung und der Einkoppelfläche der Stosswellen in den Körper des Patienten vorzusehen, wodurch die Lage des Stosswellenfokus auf den zu therapierenden Bereich im Körper des Patienten angepasst werden kann. Eine schlechtere Energieübertragung der Stosswellen aufgrund einer zusätzlichen Koppelfläche ist dabei jedoch festzustellen. Zudem sind keine stufenlos einstellbaren Eindringtiefen, sondern nur entsprechend der Dicke der Ankoppelpolster bzw. Koppelkissen fest vorgegebene Eindringtiefen möglich. Zusätzlich gestaltet sich das Handling beim Aufsetzen der Koppelaufsätze aufwendig.

Aus DE 197 18 511 C2 ist eine Vorrichtung der eingangs genannten Gattung bekannt, bei der verschiedene handgehaltene Therapieköpfe mit unterschiedlichen Eindringtiefen lösbar mit einer Versorgungseinheit verbunden werden können. Auch hier ist keine stufenlose Einstellung der Eindringtiefe möglich. Für unterschiedliche Anwendungen müssen die Therapieköpfe jeweils ausgetauscht werden.

In DE 199 35 724 A1 wird ein Therapiegerät beschrieben, bei der die Koppelmembran zur Ankopplung der Vorrichtung an den Patienten flexibel ausgebildet und durch eine Koppelflüssigkeitsverschiebung unterschiedlich weit zur Behandlungsstelle bewegt und an den Körper des Patienten angelegt werden kann. Der Abstand zwischen der Stosswellenerzeugungseinrichtung und der Koppelfläche zur Einkopplung der Stosswellen in den zu behandelnden Körper des Patienten ist somit variabel. Damit wird eine stufenlose Einstellung der Eindringtiefe der Stosswellen in den Körper des Patienten erreicht. Jedoch ist in der genannten Vorrichtung eine elastische Koppelmembran erforderlich, welche aufgrund der starken Beanspruchung einer schnellen Alterung unterliegt. Weiterhin ist zur Steuerung der Verschiebung der Koppelflüssigkeit eine Drucklufterzeugungseinrichtung mit einer entsprechenden Druckluftleitung notwendig. Eine unhandhandliche Handhabung und eine aufwendige und kostenintensive Herstellung aufgrund der zusätzlichen Drucklufterzeugungsvorrichtung erweist sich als nachteilig.

Allen vorgenannten Varianten ist gemeinsam, dass entweder die Koppelmembran verstellt wird oder Zusatzteile erforderlich sind. Dies verkürzt einerseits die Lebensdauer der Vorrichtung, andererseits gestaltet sich die Handhabung umständlich.

Vor dem Hintergrund der vorstehenden Ausführung liegt der Erfindung die Aufgabe zugrunde eine Vorrichtung der eingangs genannten Gattung so zu verbessern, dass eine hohe Flexibilität in der Anwendung eine schnelle und einfache Positionierung des Stosswellenfokus in das Therapiegebiet bei einem geringen Übertragungsverlust der Stosswellen erreicht wird.

Diese Aufgabe wird erfindungsgemäss gelöst durch eine Vorrichtung mit den Merkmalen des Anspruchs 1.

Vorteilhafte Ausführungen der Erfindung sind in den Unteransprüchen angegeben.

Der wesentliche Gedanke der Erfindung besteht darin, eine Vorrichtung zur Applikation von akustischen Stosswellen, die auf ein zu behandelndes Gebiet eines menschlichen oder tierischen Körpers gerichtet sind, bestehend aus einem Gehäuse mit wenigstens einer Koppelfläche zur Einkopplung der Stosswellen in den zu behandelnden Körper, mit einer Stosswellenerzeugungseinrichtung und einem zur Fokussierung der Stosswellen geeigneten Mittel so auszuführen, dass die Stosswellenerzeugungseinrichtung mit dem zur Fokussierung der Stosswellen geeigneten Mittel im Gehäuse relativ zur Koppelfläche beweglich ausgebildet ist.

Die Position der Stosswellenerzeugungseinrichtung zusammen mit dem zur Fokussierung der Stosswellen geeigneten Mittel ist erfindungsgemäss im Gehäuse relativ zu der Koppelfläche veränderbar. In vorteilhafter Weise ist dabei die Stosswellenerzeugungseinrichtung zusammen mit dem zur Fokussierung der Stosswellen geeigneten Mittel auf einer ersten Achse, die von der Stosswellenerzeugungseinrichtung durch die Ankoppelfläche zu einem von dem zur Fokussierung der Stosswellen geeigneten Mittel erzeugten Stosswellenfokus verläuft, bewegbar ausgebildet. Mit einer axialen Verschiebung der Stosswellenerzeugungseinrichtung zusammen mit dem zur Fokussierung der Stosswellen geeigneten Mittel der erfindungsgemässen Vorrichtung in Richtung Koppelfläche wird die Eindringtiefe der Stosswellen in den zu behandelnden Patienten variiert und kann auf das zu therapierende Gebiet eingestellt werden. Das Gehäuse mit der Koppelfläche ist gegenüber der Stosswellenerzeugungseinrichtung und dem zur Fokussierung der Stosswellen geeigneten Mittel ortsfest angeordnet.

Mit der erfindungsgemässen Vorrichtung lässt sich ohne eine Verwendung von Zubehörteilen die Eindringtiefe der Stosswellen in den Körper des Patienten und damit die Fokustiefe mit ein und derselben Vorrichtung stufenlos variieren.

Die Koppelfläche, die in bevorzugter Weise von einer Koppelmembran gebildet wird, muss nicht bewegt werden und unterliegt damit keinem Verschleiss und keiner vorzeitigen Alterung.

In einer weiteren Ausführung ist die Stosswellenerzeugungseinrichtung zusammen mit dem zur Fokussierung der Stosswellen geeigneten Mittel auf wenigstens einer zweiten Achse, die einen Winkel mit der ersten Achse bildet, bewegbar ausgebildet. Dies ermöglicht neben der Positionierung des Stosswellenfokus in ein Therapiegebiet, welches unterschiedlich weit von der Koppelfläche der Therapievorrichtung entfernt lokalisiert sein kann, zusätzlich eine Positionierung des Stosswellenfokus auf einer Fläche die beispielsweise senkrecht zur Ausbreitungsrichtung der Stosswellen in dem Körper des Patienten verläuft.

Es lässt sich damit einerseits der Stosswellenfokus schneller in das zu therapierende Gebiet im Körper des Patienten positionieren, andererseits ein grösseres Gebiet im Körper des Patienten therapieren, ohne die Notwendigkeit den Patienten, eine Patientenlagerungsvorrichtung oder die Therapievorrichtung zu bewegen. Dies ist vor allem bei der Behandlung von orthopädischen Erkrankungen von Vorteil. Insbesondere bei der Behandlung von Pseudarthrosen ist es vorteilhaft den Stosswellenfokus auf dreidimensionalen Achsen entlang einer Fraktur zu verschieben.

In einer weiteren vorteilhaften Ausführung der erfindungsgemässen Vorrichtung ist die Stosswellenerzeugungseinrichtung zusammen mit dem zur Fokussierung der Stosswellen geeigneten Mittel an einem Punkt im Gehäuse befestigt und derart bewegbar ausgebildet, dass die Stosswellenerzeugungseinrichtung zusammen mit dem zur Fokussierung der Stosswellen geeigneten Mittel um diesen Punkt kippbar ist. Dies ermöglicht eine Bewegung des Stosswellenfokus über eine kreissegmentförmige Fläche.
Die Stosswellenerzeugungseinrichtung kann zudem zusammen mit dem zur Fokussierung der Stosswellen geeigneten Mittel in Richtung Koppelfläche bewegt und gleichzeitig oder nacheinander in verschiedene Richtungen gekippt werden. Mit den beschriebenen Bewegungen lässt sich der Stosswellenfokus innerhalb eines dreidimensionalen Therapiegebietes im Körper des Patienten bewegen.

Die Bewegung der Stosswellenerzeugungseinrichtung zusammen mit dem zur Fokussierung der Stosswellen geeigneten Mittel kann sowohl manuell als auch elektro-mechanisch über einen elektrischen Antrieb erfolgen.

Die Position der Stosswellenerzeugungseinrichtung zusammen mit dem zur Fokussierung der Stosswellen geeigneten Mittel auf der ersten Achse kann erfasst und somit die Eindringtiefe der Stosswellen im Körper des Patienten und die Lage des Stosswellenfokus im Abstand von der Koppelfläche bestimmt und dem Anwender angezeigt werden. Die Anzeige kann mittels einer numerischen Skala auf der Aussenseite des Gehäuses oder über eine elektrische Anzeige erfolgen.

In der Ausführung der erfindungsgemässen Vorrichtung bei der die Bewegung der Stosswellenerzeugungseinrichtung mit dem zur Fokussierung der Stosswellen geeigneten Mittel mittels eines elektro-mechanischen Antriebs erfolgt, kann die Umdrehung des Antriebsmotors erfasst, die erfolgte Lageänderung berechnet und die Lage des Stosswellenfokus im Abstand von der Koppelfläche an einer Anzeigeeinheit der Therapievorrichtung zur Anzeige gebracht werden.
Dies gestattet eine schnelle Behandlungspositionierung der Therapievorrichtung bei Behandlungsstellen, die sich im hautnahen Bereich des Patienten befinden.

Ferner ist in einer weiteren Ausführung am Gehäuse der Therapievorrichtung eine Bedieneinheit für die elektromechanische Bewegung der Stosswellenerzeugungseinrichtung mit dem zur Fokussierung der Stosswellen geeigneten Mittel und damit zur Positionierung des Stosswellenfokus in das Therapiegebiet vorgesehen. Hierdurch ist die Handlichkeit der Therapievorrichtung weiter gesteigert.

Insbesondere kann der Bewegungablauf der Stosswellenerzeugungseinrichtung und dem zur Fokussierung der Stosswellen geeigneten Mittel mit einem bildgebenden System gekoppelt werden. Hierbei kann die Bewegung der Stosswellenerzeugungseinrichtung mit dem zur Fokussierung der Stosswellen geeigneten Mittel zur Positionierung des Stosswellenfokus in das entsprechende Therapiegebiet nach erfolgter Lokalisation mit dem bildgebenden System vorzugsweise automatisch erfolgen.

Mit der erfindungsgemässen Lösung sind neben einer verbesserten Handhabung und Verschleissfestigkeit die Flexibilität der Vorrichtung in Form einer Erhöhung der Einsatzmöglichkeiten deutlich gestiegen.
Die Stosswellenerzeugung der Stosswellenerzeugungseinrichtung beruht in einer bevorzugten Ausführung auf dem elektrohydraulischen Prinzip. Die Erzeugung der Stosswellen kann aber auch beispielsweise elektromagnetisch oder piezoelektrisch erfolgen.

Die Fokussierung wird in an sich bekannter Weise mit einem Reflektor realisiert. Im Falle der Fokussierung der Stosswellen durch Reflektoren können diese als halbschalenförmige Ellipsoiden oder Paraboloiden mit verschiedenen Achsverhältnissen ausgebildet sein. Zur Fokussierung können aber auch akustische Linsen mit unterschiedlicher Brennweite verwendet werden. Weiter können halbschalenförmige Anordnungen von elektrischen Membranen oder Piezokristallelementen mit unterschiedlichen Durchmessern verwendet werden.
Mit einer unterschiedlichen geometrischen Ausbildung der zur Fokussierung der Stosswellen geeigneten Mittel wird die maximale Eindringtiefe der Stosswellen definiert.

Im Folgenden wird die Erfindung anhand von in den Zeichnungen dargestellten Ausführungsbeispielen näher erläutert. Es zeigen:
- Figur 1: Schnittbild einer Seitenansicht der erfindungsgemässen Vorrichtung in einer ersten Ausführung,
- Figur 2: Schnittbild einer Seitenansicht der erfindungsgemässen Vorrichtung in einer zweiten Ausführung
- Figur 3: Schnittbild einer Seitenansicht der erfindungsgemässen Vorrichtung in einer dritten Ausführung und
- Figur 4: Schnittbild einer Seitenansicht der erfindungsgemässen Vorrichtung in einer Ausführung mit dreidimensionaler Bewegung.

In den Zeichnungen sind vier Ausführungsbeispiele der erfindungsgemässen Vorrichtung schematisch und vereinfacht dargestellt.

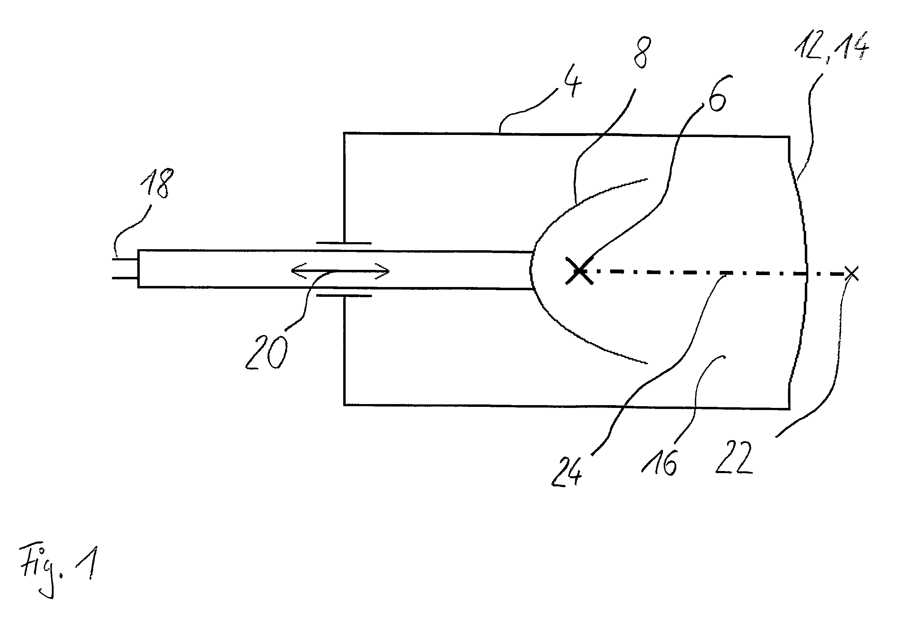

Das in Figur 1 dargestellte Schnittbild zeigt die erfindungsgemässe Vorrichtung in einer ersten Ausführung in einer Seitenansicht. Ein Gehäuse 4 bildet mit einer Koppelmembran 14 ein abgeschlossenes Volumen, indem ein für die Fortleitung der Stosswellen geeignetes Medium 16 eingebracht ist. Die Koppelmembran 14 bildet in der dargestellten Ausführung die Koppelfläche 12 der Therapievorrichtung zur Einkopplung der Stosswellen in den zu behandelnden Körper.

In dem Gehäuse 4 befinden sich eine Stosswellenerzeugungseinrichtung 6, die über wenigstens eine Versorgungsleitung 18 betrieben wird und ein für die Fokussierung der Stosswellen geeignetes Mittel 8, im folgenden Fokussierungseinrichtung 8 genannt.

Die Stosswellenerzeugungseinrichtung 6 und die Fokussierungseinrichtung 8 sind gemeinsam auf einer ersten Achse 24 beweglich angeordnet, welche von der Stosswellenerzeugungseinrichtung 6 durch die Koppelmembran 14 zu einem von der Fokussierungseinrichtung 8 erzeugten Stosswellenfokus 22 verläuft. Eine axiale Bewegung 20 der Stosswellenerzeugungseinrichtung 6 zusammen mit der Fokussiereinrichtung 8 erfolgt bezogen auf das ortsfeste Gehäuse 4 in und entgegen der Richtung zur Koppelfläche 12. Der Abstand der Stosswellenerzeugungseinrichtung 6 mit der Fokussierungseinrichtung 8 zur Koppelfläche 12 kann variiert werden. Der Abstand der Stosswellenerzeugungseinrichtung 6 zu dem Stosswellenfokus 22 wird durch die Geometrie der Fokussierungseinrichtung 8 bestimmt. Durch die Variierung des Abstandes der Fokussierungseinrichtung 8 zusammen mit der Stosswellenerzeugungseinrichtung 6 zur Koppelfläche 12 wird die Eindringtiefe der Stosswellen im Körper des Patienten und damit die Lage des Stosswellenfokus 22 verändert.

Eine Bewegung 20 der Stosswellenerzeugungseinrichtung 6 zusammen mit der Fokussierungseinrichtung 8 im Gehäuse 4 kann manuell, elektro-mechanisch oder hydraulisch mittels eines elektrischen bzw. hydraulischen Antriebs erfolgen.

Das Wirkprinzip der Stosswellenerzeugungseinrichtung 6 basiert in dem dargestellten Ausführungsbeispiel auf dem elektrohydraulischen Prinzip.

In einer weiteren Ausführung, die hier nicht explizit dargestellt ist, bildet unter Anwendung dieses Wirkprinzips die Stosswellenerzeugungseinrichtung 6 und die Fokussierungseinrichtung 8 mit einer zweiten Membran ein zweites abgeschlossenes Volumen, welches sich innerhalb des Volumens befindet, das durch das Gehäuse 4 und die Koppelmembran 14 erzeugt wird. In dieses zweite Volumen können leitfähige Partikel zur Begünstigung des Funkenüberschlages eingebracht werden, wie aus EP 0 781 447 B1 bekannt.

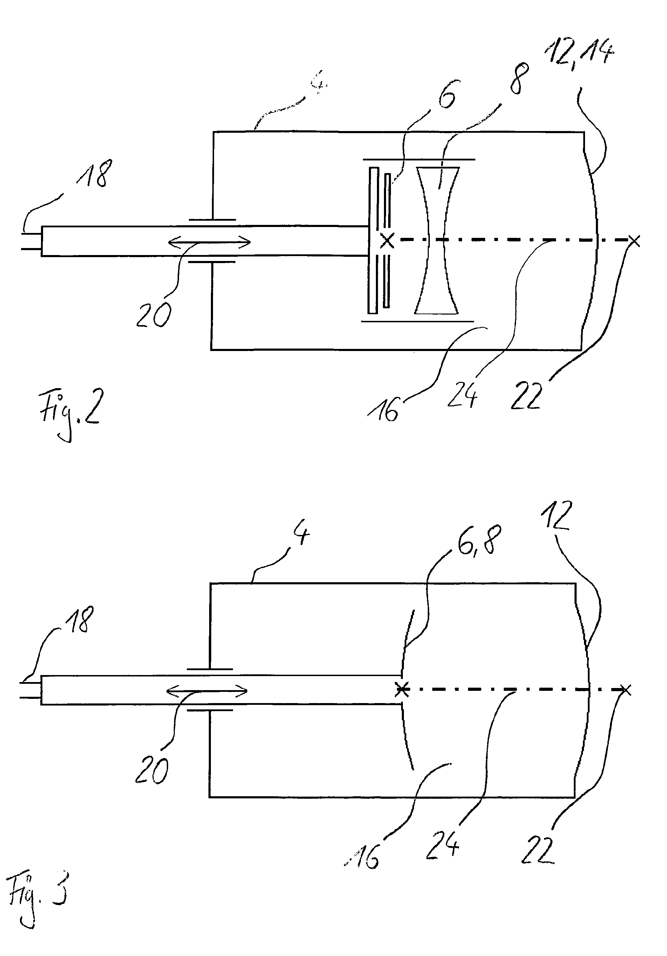

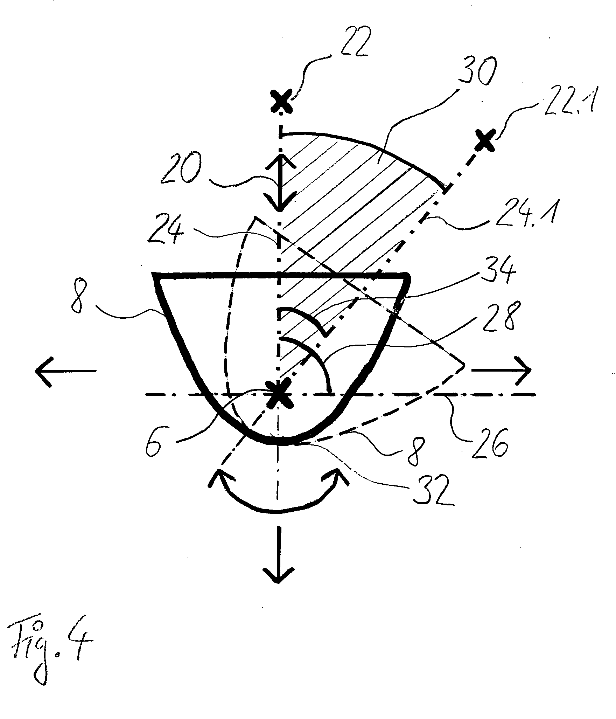

Die Stosswellen können aber auch in anderer Weise erzeugt werden, wie beispielsweise mit einer elektromagnetischen oder piezoelektrischen Stosswellenerzeugungseinrichtung 6, die in einer jeweils weiteren Ausführungsform der Erfindung in den Figuren 2 und 3 dargestellt sind.

In Figur 2 bildet eine Membran einer Flachspule die Stosswellenerzeugungseinrichtung 6. Die generierten Stosswellen werden mittels einer akustischen Linse 8 fokussiert. In dem Ausführungsbeispiel der Figur 3 werden die Stosswellen mittels kalottenförmig angeordneten Piezoelementen erzeugt. Die Form der Stosswellenerzeugungseinrichtung 6 bildet hierbei die Fokussierungseinrichtung 8.

Verschiedene Verstell- bzw. Bewegungsmöglichkeiten der erfindungsgemässen Vorrichtung sind in Figur 4 schematisch dargestellt. Eine vorteilhafte Ausgestaltung der erfindungsgemässen Vorrichtung besteht darin, dass in dem Gehäuse 4, welches in der Abbildung nicht explizit dargestellt ist, die Stosswellenerzeugungseinrichtung 6 mit der Fokussierungseinrichtung 8 zusätzlich zu der Bewegung 20 auf der ersten Achse 24 auf einer zweiten Achse 26 gleichzeitig oder nacheinander bewegbar ist. Damit kann in vorteilhafter Weise der Stosswellenfokus 22 in das zu therapierende Gebiet positioniert werden ohne einen Patienten oder, falls erforderlich, eine Patientenlagerungsvorrichtung oder die Therapievorrichtung zu bewegen. Die zweite Achse 26 bildet mit der ersten Achse 24 einen Winkel 28, der vorzugsweise 90° beträgt.

In einer weiteren vorteilhaften Ausführung ist die Stosswellenerzeugungseinrichtung 6 zusammen mit der Fokussierungseinrichtung 8 an einem Punkt 32 im Gehäuse 4 befestigt und derart bewegbar ausgebildet, dass die Stosswellenerzeugungseinrichtung 6 zusammen mit der Fokussierungseinrichtung 8 um diesen Punkt 32 kippbar ist. Die erste Achse 24 bildet mit der in der gekippter Position der Stosswellenerzeugungseinrichtung 6 und der Fokussierungseinrichtung 8 ersten Achse 24.1 einen Kippwinkel 34, um dessen Grösse die Stosswellenerzeugungseinrichtung 6 zusammen mit der Fokussierungseinrichtung 8 gekippt worden ist. Der Kippwinkel 34 kann dem Anwender angezeigt werden.
Die Hauptrichtung der Stosswellenausbreitung erfolgt damit nicht nur in einer einzigen Richtung sondern vielmehr in verschiedenen Richtungen die eine kreissegmentförmige Fläche 30 beschreiben. Der Stosswellenfokus 22 wird entlang des Umfanges der kreissegmentförmigen Fläche 30 bewegt.

Die Stosswellenerzeugungseinrichtung 6 kann zudem zusammen mit der Fokussierungseinrichtung 8 in Richtung Koppelfläche 12 bewegt und gleichzeitig oder nacheinander in verschiedene Richtungen gekippt und oder seitwärts bewegt werden. Mit den beschriebenen Bewegungen lässt sich der Stosswellenfokus 22 innerhalb eines dreidimensionalen Therapiegebietes im Körper des Patienten bewegen.

### Bezugszeichenliste

- 4: Gehäuse
- 6: Stosswellenerzeugungseinrichtung
- 8: Für die Fokussierung der Stosswellen geeigneten Mittel; Fokussierungseinrichtung
- 12: Koppelfläche
- 14: Koppelmembran
- 16: Für die Ausbreitung der Stosswelle geeignetes Medium
- 18: Versorgungsleitungen
- 20: Axiale Bewegung der Stosswellenerzeugungseinheit zusammen mit der Fokussierungseinrichtung
- 22: Stosswellenfokus
- 22.1: Stosswellenfokus in gekippter Position der Stosswellenerzeugungseinrichtung und der Fokussierungseinrichtung
- 24: Erste Achse
- 24.1: Erste Achse in gekippter Position der Stosswellenerzeugungseinrichtung und der Fokussierungseinrichtung
- 26: Zweite Achse
- 28: Winkel zwischen erster und zweiter Achse
- 30: Kreissegmentförmige Fläche
- 32: Kipppunkt
- 34: Kippwinkel

## Patentansprüche

1. Vorrichtung, zur Applikation von akustischen Stosswellen, die auf ein zu behandelndes Gebiet eines menschlichen oder tierischen Körpers gerichtet sind, bestehend aus einem Gehäuse mit einer Koppelfläche für die Einkopplung der Stosswellen in den zu behandelnden Körper, mit einer Stosswellenerzeugungseinrichtung und einem zur Fokussierung der Stosswellen geeigneten Mittel,
**dadurch gekennzeichnet, dass** die Stosswellenerzeugungseinrichtung (6) mit dem zur Fokussierung der Stosswellen geeigneten Mittel (8) im Gehäuse (4) relativ zur Koppelfläche (12) beweglich ausgebildet ist.

2. Vorrichtung nach Anspruch 1,
**dadurch gekennzeichnet, dass** die Stosswellenerzeugungseinrichtung (6) mit dem zur Fokussierung der Stosswellen geeigneten Mittel (8) auf einer ersten Achse (24), die von der Stosswellenerzeugungseinrichtung (6) durch die Koppelfläche (12) zu einem von dem zur Fokussierung der Stosswellen geeigneten Mittel (8) erzeugten Stosswellenfokus (22) verläuft, bewegbar ausgebildet ist.

3. Vorrichtung nach Anspruch 2,
**dadurch gekennzeichnet, dass** die Stosswellenerzeugungseinrichtung (6) mit dem zur Fokussierung der Stosswellen geeigneten Mittel (8) auf wenigstens einer zweiten Achse (26), die einen Winkel (28) mit der ersten Achse (24) bildet, bewegbar ausgebildet ist.

4. Vorrichtung nach einem oder mehreren der vorhergehenden Ansprüche,
**dadurch gekennzeichnet, dass** die Stosswellenerzeugungseinrichtung (6) mit dem zur Fokussierung der Stosswellen geeigneten Mittel (8) an einem Punkt (32) im Gehäuse (4) befestigt und derart bewegbar ausgebildet ist, dass die Stosswellenerzeugungseinrichtung (6) mit dem zur Fokussierung der Stosswellen geeigneten Mittel (8) um diesen Punkt (32) kippbar ist.

5. Vorrichtung nach Anspruch 4,
**dadurch gekennzeichnet, dass** ein Winkel (34), um den die Stosswellenerzeugungseinrichtung (6) mit dem zur Fokussierung der Stosswellen geeigneten Mittel (8) gekippt wird, angezeigt wird.

6. Vorrichtung nach einem oder mehreren der vorhergehenden Ansprüche,
**dadurch gekennzeichnet, dass** die Bewegung der Stosswellenerzeugungseinrichtung (6) mit dem zur Fokussierung der Stosswellen geeigneten Mittel (8) manuell erfolgt.

7. Vorrichtung nach einem oder mehreren der vorhergehenden Ansprüche,
**dadurch gekennzeichnet, dass** die Bewegung der Stosswellenerzeugungseinrichtung (6) mit dem zur Fokussierung der Stosswellen geeigneten Mittel (8) elektro-mechanisch erfolgt.

8. Vorrichtung nach Anspruch 7,
**dadurch gekennzeichnet, dass** die Bewegung der Stosswellenerzeugungseinrichtung (6) mit dem zur Fokussierung der Stosswellen geeigneten Mittel (8) von einem bildgebenden System bestimmt wird.

9. Vorrichtung nach Anspruch 1,
**dadurch gekennzeichnet, dass** die Stosswellenerzeugung der Stosswellenerzeugungseinrichtung (6) vorzugsweise auf dem elektrohydraulischen Prinzip beruht.

10. Vorrichtung nach einem oder mehreren der vorhergehenden Ansprüche,
**dadurch gekennzeichnet, dass** die Position der Stosswellenerzeugungseinrichtung (6) mit dem zur Fokussierung der Stosswellen geeigneten Mittel (8) auf der ersten Achse (24) direkt oder indirekt angezeigt wird.
